# EUROPÄISCHE PATENTSCHRIFT

(11) **EP 3 867 896 B1**
(45) Veröffentlichungstag und Bekanntmachung des Hinweises auf die Patenterteilung: **31.05.2023**
(21) Anmeldenummer: 18799446.2
(22) Anmeldetag: 17.10.2018
(51) Int. Cl.: G09B 19/00, G09B 5/02, G06V 40/20, A61B 5/024, A61B 5/11, A61B 5/22, A61B 5/00

(54) **TRAININGSMODUL**
TRAINING MODULE
MODULE D'ENTRAÎNEMENT

(43) Veröffentlichungstag der Anmeldung: 25.08.2021
(73) Patentinhaber: Sphery AG, 9434 Au SG (CH)
(72) Erfinder: MARTIN-NIEDECKEN, Anna Lisa, 8700 Küsnacht (CH)
(74) Vertreter: Troesch Scheidegger Werner AG
(86) Internationale Anmeldenummer: PCT/EP2018/078417
(87) Internationale Veröffentlichungsnummer: WO 2020/078546

(56) Entgegenhaltungen:
- DE-A1-102012 101 152
- DE-A1-102017 102 144
- US-A1- 2013 004 928
- US-A1- 2013 171 601
- US-B1- 8 795 138

## Beschreibung

### TECHNISCHES GEBIET

Die vorliegende Erfindung betrifft ein Trainingsmodul, insbesondere ein interaktives Trainingsmodul für den menschlichen Körper.

### STAND DER TECHNIK

Um den Körper zu trainieren begeben sich viele Leute in ein Fitnessstudio, wo sie von einem Instruktor betreute Übungen ausführen können. Die Übungen können im Gruppenunterricht oder im Einzelunterricht ausgeführt werden, welche zu vorgegebenen Zeiten stattfinden. Dementsprechend ist es nicht möglich zu einer beliebigen Zeit zu trainieren. Um die Flexibilität und die Individualität des Trainings zu erhöhen werden Instruktionsvideos angeboten. Diese können beispielsweise zu Hause an einem Bildschirm selbstständig abgespielt werden. Die Übungen werden am Bildschirm angezeigt und über die Lautsprecher werden entsprechende Instruktionen abgegeben. Ein Benutzer eines solchen Instruktionsvideos hat keine Möglichkeit zu erkennen, ob er die Übungen, bzw. Instruktionen korrekt ausführt. Auch ist es so, dass eine Übung vorab ausgewählt werden muss und diese dann automatisch abläuft, ungeachtet der Ausübung der Übung durch den Benutzer. Entspricht die ausgewählte Übung nicht dem Leistungsniveau des Benutzers, so muss dieser manuell eine andere Übung auswählen.

Die US 2013/0004928 A1 offenbart ein Trainingsmodul mit mehreren aneinander anschliessenden bildanzeigenden Wänden, bei welchem mittels Projektoren Bilder auf die Wände projiziert werden, wobei die Projektoren an einer von den Wänden unabhängigen Konstruktion angeordnet sind.

Die DE 10 2012 101 152 A1, die DE 10 2017 102 144 A1 und die US 2013/0171601 A1 offenbaren Trainingsmodule mit einer einzigen bildanzeigenden Wand.

### BESCHREIBUNG DER ERFINDUNG

Eine Aufgabe der vorliegenden Erfindung besteht darin, ein Trainingsmodul bereitzustellen, welches das unabhängige Trainieren ohne einen physischen Instruktor ermöglicht, bei einer hohen und auf den Benutzer angepassten Trainingsqualität.

Diese Aufgabe wird durch ein Trainingsmodul mit den Merkmalen des Anspruchs 1 gelöst. Weitere Ausführungsformen des Trainingsmoduls, eines Trainingssystems, sowie eines Verfahrens zum Betreiben eines Trainingsmoduls sind durch die Merkmale von weiteren Ansprüchen definiert.

Ein erfindungsgemässes interaktives Trainingsmodul umfasst mindestens eine bildanzeigende Wand, mit welcher einem Benutzer Bewegungsabläufe und/oder Objekte angezeigt werden können, mindestens eine Positionserfassung, mit welcher die Positionen zumindest der Hände des Benutzers erfassbar sind und eine Steuereinheit, mit welcher diese Positionen mit denen verglichen werden kann, welche in der Steuereinheit hinterlegt sind, wobei die Steuereinheit aufgrund der Differenz zwischen der hinterlegten Position und der erfassten Position dem Benutzer mittels der mindestens einen bildanzeigenden Wand Korrekturangaben anzeigen kann.

Ein solches Trainingsmodul erlaubt das selbstständige Trainieren mit einem optimalen Trainingseffekt. Durch die kontinuierliche Erfassung der Position zumindest der Hände, kann deren Bewegung mittels der Steuereinheit ermittelt werden. Die Positionserfassung, bzw. die Bewegungserfassung kann auch in Intervallen erfolgen, deren Zeitabstände einstellbar sind; Beispielsweise kurze Zeitabstände bei schnellen Übungen und längere Zeitabstände bei langsameren Übungen.

Die Bewegungsabläufe können Abläufe für nur einzelne Gliedmassen sein oder können den ganzen Körper betreffen. Beispielsweise können einzelne Übungen wie Liegestützen, Kniebeugen oder dergleichen angezeigt werden oder es können mehrere hintereinander auszuführende Abläufe angezeigt werden, wie beispielsweise bei Yoga, Tai-Chi oder dergleichen. Ein solches Trainingsmodul kann ein breites Spektrum an Anforderungen abdecken, beispielsweise im Bereich Rehabilitation und/oder Altenpflege.

Das Trainingsmodul bietet die Möglichkeit der Vordefinition von Spielertypen, welche sich beispielsweise durch ihre körperlichen und geistigen Leistungsfähigkeiten unterscheiden. Der Benutzer kann einen Spielertyp auswählen und das System vergleicht die Vorgaben mit den vom Benutzer ausgeführten Bewegungen. Alternativ kann das System verlangen, dass der Benutzer ein Tutorial absolviert, welches dem System die Zuordnung zu einem Spielertyp erlaubt. Dies kann vor jedem Training zur Überprüfung der aktuellen Leistungsfähigkeit erfolgen. Alternativ kann dies nur erstmalig erfolgen oder das System kann den Benutzer dazu auffordern, wenn sein letztes Training schon lange her ist. Das System kann einen anderen Spielertyp vorschlagen, wenn erkannt wird, dass die vom Benutzer ausgeführten Bewegungen zu stark von den Vorgaben abweichen. Das Trainingsmodul bietet daher eine optimal an den Benutzer angepasste Forderung und Förderung und somit ausgewogenes und maximal effektives Training sicher. Die Anpassung an die individuellen Bedürfnisse kann in Echtzeit erfolgen.

Alternativ oder zusätzlich können Bewegungsvorgaben in der Form von Objekten angezeigt werden, die dem Benutzer anzeigen, in welche Richtung er welche Gliedmassen bewegen soll. Beispielsweise kann eine Route angezeigt werden, welcher der Benutzer folgen soll. Durch die Bewegung der Gliedmassen kann der Benutzer seine Bewegungsrichtung anzeigen oder ändern. Die Route kann Hindernisse beinhalten, welche den Benutzer auffordern, auszuweichen, hochzuspringen oder sich zu ducken. Durch ein optisches Feed-Back an der Wand kann dem Benutzer angezeigt werden, wie gut er die vorgegebene Route nachvollzogen hat.

In einer Ausführungsform umfasst die Positionserfassung mindestens eine Kamera, mit welchem der Bereich vor der Wand erfassbar ist, wodurch die Bewegungen eines Benutzers vor der Wand erfassbar sind. Es werden alle Gelenke am Körper mit einer Kamera oder einer vergleichbaren Technologie getrackt, d.h. deren Bewegung aufgezeichnet und ausgewertet. Dies ermöglicht einen sehr genauen Vergleich der Trainings-Vorgabe mit der tatsächlich ausgeführten Bewegung, was eine qualitativ hochstehende Rückmeldung bezüglich der Bewegungsausführung erlaubt. Diese Informationen können auch zur Instruktion der Verbesserung der Bewegungen beim nächsten Versuch verwendet werden. Dementsprechend kann das Training optimiert werden und es können Verletzungen, beispielsweise durch eine Überlastung vermieden werden.

Der Benutzer kann spezielle Kleidung tragen oder kann Markierungen tragen, die das Erkennen der Bewegungen mittels der Kamera verbessern. Alternativ oder zusätzlich umfasst die Positionserfassung mindestens einen induktiven, kapazitiven oder Infrarotlicht erfassenden Sensor, mit welchem der Bereich vor der Wand erfassbar ist. Beispielsweise kann der Sensor das Licht eines Infrarot-Lasers erfassen.

In einer Ausführungsform umfasst das interaktive Trainingsmodul mindestens einen Lautsprecher, mit welchem Instruktionen gegeben werden können. Beispielsweise kann die zu absolvierende Übung erklärt werden. Alternativ oder zusätzlich können akustische Korrekturangaben gemacht werden. Beispielsweise kann der Benutzer aufgefordert werden schneller zu reagieren oder sich genauer zu bewegen. Alternativ oder zusätzlich können motivierende oder animierende Kommentare oder Musik abgegeben werden. Beispielsweise kann der Benutzer aufgefordert werden, noch etwas länger durchzuhalten oder es kann Musik abgespielt werden, welche zur Intensität der zu absolvierenden Übung passt. Die Lautsprecher können auch zur Kommunikation mit anderen Benutzern verwendet werden. In diesem Fall umfasst das Trainingsmodul auch mindestens ein Mikrofon.

In einer Ausführungsform umfasst das interaktive Trainingsmodul mindestens einen Herzrhythmus-Sensor, mit welchem der Herzrhythmus des Benutzers kontinuierlich und/oder in Intervallen erfassbar ist. Beispielsweise kann vor Übungsbeginn der Ruhepuls erfasst werden. Das Trainingsmodul schlägt dann eine Übung vor, welche auf den erfassten Ruhepuls abgestimmt ist. Es können weitere Angaben vor Übungsbeginn erfasst werden, welche anschliessend zur optimalen Übungsauswahl, bzw. dem optimalen Vorschlag einer Übung durch das Trainingsmodul genutzt werden können. Beispielsweise kann das Alter, das Gewicht und die allgemeine Fitness eingegeben werden. Zudem können Präferenzen bezüglich der optischen Darstellung der Übungen auf der Wand angegeben werden. Beispielsweise ist eine technische Darstellung oder eine natürliche Darstellung auswählbar.

In einer Ausführungsform verwendet die Steuereinheit den erfassten Herzrhythmus, um den Schwierigkeitsgrad und/oder die Intensität des Trainingsprogramms anzupassen. Die Anpassung kann während der Übung erfolgen oder sie kann danach erfolgen. Beispielsweise kann die Anpassung in Echtzeit und ohne das Eingreifen eines Instruktors oder Trainers erfolgen, wodurch der Benutzer immer in seinem optimalen Trainingszustand gehalten wird. Wird beispielsweise währen einer Übung festgestellt, dass der Herzrhythmus, bzw. der Puls des Benutzers sehr hoch ist, so kann beispielsweise die Geschwindigkeit reduziert werden, mit welcher die vorgegebenen Bewegungen auszuführen sind oder die Intensität der Bewegungen kann reduziert werden. Zum Beispiel kann die Intensität der Übung verkleinert werden, d.h. die Grösse der Bewegungen kann verkleinert werden. Alternativ oder zusätzlich können die zeitlichen Abstände zwischen den zu absolvierenden Übungen vergrössert werden. Umgekehrt können die Bewegungen vergrössert werden und die zeitlichen Abstände können verkleinert werden, wenn die Übungen intensiviert werden sollen.

In einer Ausführungsform umfasst das interaktive Trainingsmodul einen Kraftsensor, welcher in der mindestens einen bildanzeigenden Wand integriert ist. Die Wand kann auch mehrere integrierte Kraftsensoren umfassen, welche gleichmässig oder beliebig über die Fläche verteilt angeordnet sind. Mittels der Kraftsensoren kann die Steuereinheit den Ort und die Intensität einer Berührung mit der Wand erfassen. Die Steuereinheit kann aufgrund der Auswertung der erfassten Daten dem Benutzer an der Wand anzeigen, ob seine Berührungen zu ungenau, zu stark oder zu schwach waren oder zu einem falschen Zeitpunkt erfolgt sind.

In einer Ausführungsform ist die mindestens eine bildanzeigende Wand ein Bildschirm.

Erfindungsgemäss umfasst das interaktive Trainingsmodul mindestens einen Projektor mit welchem ein Bild auf der bildanzeigenden Wand anzeigbar ist. Ein angezeigtes Bild kann einen Teil, Teile oder die gesamte Wand bedecken. Beispielsweise ist die Projektion eines 150 Zoll Bildes in einen Abstand von 50 Centimetern mit einem Ultra Short Distance Laser Projektor möglich. Solche Projektoren zeichnen sich durch einen sehr hohen Kontrastwert aus. Somit kann auch bei schlechten Lichtverhältnissen ein qualitativ hochstehendes Bild projiziert werden.

In einer Ausführungsform umfasst die mindestens eine bildanzeigende Wand eine tragende erste Schicht und eine haptische zweite Schicht, wobei die haptische Schicht auf der gegen den Benutzer gerichteten Seite der tragenden Schicht angeordnet ist.

In einer Ausführungsform umfasst die erste Schicht eine MDF-Platte und die zweite Schicht umfasst eine Schaumstoff-Platte, eine Silikon-Platte oder eine Silikon-Schaumstoff-Platte, welche mit der MDF-Platte verleimt ist.

In einer Ausführungsform umfasst die mindestens eine bildanzeigende Wand eine optische dritte Schicht, wobei die optische Schicht auf der gegen den Benutzer gerichteten Seite der haptischen Schicht angeordnet ist.

In einer Ausführungsform umfasst die dritte Schicht eine Leinwand ist, welche zumindest über die Schaumstoff- oder Silikon-Platte gespannt ist.

Erfindungsgemäss umfasst das interaktive Trainingsmodul eine erste Wand und mindestens zwei in der bestimmungsgemässen Gebrauchslage an diese auf beiden Seiten seitlich anschliessende zweite Wände, wobei die mindestens zwei zweiten Wände winklig zur ersten Wand ausgerichtet sind. Die erste Wand und die mindestens zwei zweiten Wände schliessen jeweils auf der gegen den Benutzer gerichteten Seite einen Winkel von 90 Grad oder mehr ein. Beispielsweise einen Winkel von 110 Grad. Die zweiten Wände können gelenkig mit der ersten Wand verbunden sein, wodurch der Winkel, welcher durch die beiden Wände aufgespannt wird, einstellbar ist. Alternativ kann der Winkel jedoch auch weniger als 90 Grad betragen.

Es können weitere Wände vorgesehen sein, beispielsweise können insgesamt vier, fünf, sechs oder mehr Wände vorgesehen sein, welche zusammen einen offenen oder einen geschlossenen Bereich bilden. Bei einem geschlossenen Bereich kann eine Türe vorgesehen sein oder eine der Wände kann schwenkbar oder verschiebbar sein. Zusätzlich zu den Seitenwänden kann eine Decke vorgesehen sein, welche ebenfalls als bildanzeigende Wand dienen kann und ebenfalls integrierte Kraftsensoren umfassen kann. Alternativ können die offenen Bereiche des Trainingsmoduls mit einem Vorhang oder einem Rouleau verschlossen werden. Dies gilt für die Seite, d.h. die Wände, sowie auch für die Decke.

In einer Ausführungsform umfassen die Wände mindestens ein Paneel und einen Teil eines Eckstücks, welches die erste Wand im Wesentlichen übergangslos mit der zweiten Wand verbindet. Das Eckstück kann auch scharfkantig ausgeführt sein. Ebenfalls ist es denkbar, dass eine Wand mehrere Paneele umfasst, welche stumpf aneinandergereiht sind. In einer alternativen Ausführungsform ohne Eckstück können zwei benachbarte Paneele winklig zueinander anschliessend aneinander angeordnet werden. Es ist ebenfalls möglich, zwei benachbarte Paneele gelenkig miteinander zu verbinden, wodurch die Paneele unter einem beliebigen Winkel zueinander anordbar sind. Die Paneele einer Wand können auch zumindest teilweise versetzt zueinander auf Schienen angeordnet sein, wodurch die Breite der Wand leicht einstellbar ist. Ebenfalls kann eine oder beide Seitenwände verschiebbar ausgestaltet sein, wodurch der Raum zwischen den Wänden leicht veränderbar ist. Ein Paneel kann eine oder mehrere Platten umfassen, welche vertikal und/oder horizontal neben, bzw. übereinander angeordnet sind.

Erfindungsgemäss umfasst das interaktive Trainingsmodul drei oder mehr Projektoren, wobei auf jeder der Wände mit mindestens einem der Projektoren ein Bild anzeigbar ist. Es können auch einzelne oder mehrere Teilbilder angezeigt werden. Alternativ oder zusätzlich kann ein Projektor ein Bild auf zwei zueinander benachbarten Wänden anzeigen.

Erfindungsgemäss umfasst das interaktive Trainingsmodul ein Gerüst, an welchem zumindest die Wände angeordnet sind. Das Gerüst kann freistehend im Raum angeordnet sein oder kann mit dem Boden, den Wänden oder der Decke fest verbunden sein. Das Gerüst ragt in der bestimmungsgemässen Gebrauchslage nach oben über die Wände und die Projektoren sind an dem über die Wände ragenden Teil des Gerüsts angeordnet. Die Projektoren können direkt am Gerüst angeordnet sein oder sie können mit einer Halterung, beispielsweise einer Halteplatte am Gerüst angeordnet sein. Die Halteplatte kann an einem oder mehreren Trägern des Gerüsts befestigt sein.

In einer Ausführungsform ist die Positionserfassung an dem über die Wände ragenden Teil des Gerüsts angeordnet. Alternativ kann die Positionserfassung an einer über dem Trainingsmodul befindlichen Decke angeordnet sein.

Ebenfalls ist es möglich, die Positionserfassung in die Wände zu integrieren.

In einer Ausführungsform sind die über die Wände ragenden Teile des Gerüsts miteinander verbunden. Die Verbindung kann teilweise oder vollständig über Profile erfolgen. Beispielsweise können die Profilteile mit einem Deckensegment miteinander verbunden sein. Beispielsweise kann eine Wand eine Einheit aus einem Gerüst einem oder mehreren Paneelen, einem oder mehrerer Projektoren und einer oder mehreren Positionserfassungen sein. Die Projektoren und Positionserfassungen können auf die entsprechende Wand voreingestellt sein. Mehrere solcher Einheiten können dann miteinander ausgerichtet und miteinander verbunden werden. Die Anzahl der Wände, deren Position zueinander, d.h. deren Reihenfolge und deren Ausrichtung können in der Systemsteuerung eingegeben werden, was eine einfache und vielseitige Installation erlaubt.

In einer Ausführungsform umfasst das interaktive Trainingsmodul einen Boden, welcher bildanzeigend ausgestaltet sein kann und/oder welcher mit Kraftsensoren bestückt ist. Die Kraftsensoren können gleichmässig oder beliebig über die Bodenfläche verteilt angeordnet sein.

In einer Ausführungsform umfasst das interaktive Trainingsmodul mindestens einen Projektor, mit welchem ein Bild auf dem Boden anzeigbar ist. Es können auch zwei oder mehr Projektoren vorgesehen sein, welche ein Bild auf den Boden projizieren können. Alternativ oder zusätzlich können diese Projektoren auch Bilder oder Teilbilder auf die Wände projizieren. Der Boden kann als interaktives Eingabeelement dienen. Er dient ebenfalls als Orientierungshilfe für die zeitlich, richtige Ausführung der verlangten Bewegungen. Die Orientierungshilfe wird mit Hilfe von Audio-visuellen Effekten erreicht, welche die angesagte Bewegung auf dem Boden in Echtzeit anzeigen. Der Boden kann zudem die Startposition zeigen, welche je nach Größe, geistiger und physischer Verfassung des jeweiligen Benutzers variieren kann.

Die erwähnten Ausführungsformen des Trainingsmoduls lassen sich in beliebiger Kombination einsetzen, sofern sie sich nicht widersprechen.

Ein erfindungsgemässes interaktives Trainingssystem umfasst ein Trainingsmodul gemäss einem der vorangehenden Ausführungsformen und mindestens einen Tracker, welcher von einem Benutzer tragbar ist und mit welchem zumindest die Position einer Hand des Benutzers erfassbar ist. Ein solcher Tracker kann beispielsweise ein Infrarotsignal aussenden oder empfangen.

In einer Ausführungsform umfasst das interaktive Trainingssystem zwei oder mehr Tracker, welche mit den Händen gehalten werden können oder welche mit Befestigungsmitteln am Körper des Benutzers angeordnet werden können.

In einer Ausführungsform sind die Befestigungsmittel ausgebildet, um die Tracker an mindestens einer der Gruppe von Körperstellen anbringen zu können, welche Handgelenk, Ellbogen, Schulter, Nacken, Kopf, Hüfte, Knie und Fussgelenk umfasst.

In einer Ausführungsform ist das Trainingssystem derart ausgebildet, dass die Bewegungen von zwei oder mehr Benutzern erfassbar sind. Hierfür wird eine entsprechende Anzahl an Positionserfassungen im System bereitgestellt und die Steuereinheit ist entsprechend ausgestaltet. Durch die Bereitstellung der benötigten Anzahl von Positionserfassungen und die dementsprechende Programmierung, bzw. Ausgestaltung der Steuereinheit sind auch die nachfolgenden Ausführungsformen realisierbar.

In einer Ausführungsform kann die Erfassung gleichzeitig erfolgen. Alternativ kann die Erfassung zeitversetzt erfolgen. Bei der zeitversetzten Variante kann ein Benutzer gegen sich selbst antreten oder er kann gegen die aufgezeichnete Übung eines anderen antreten. Das System vergleicht dann die beiden Benutzer miteinander und mit der Vorgabe. Die Benutzer können dementsprechend eine Übung gemeinsam ausführen oder sie können gegeneinander antreten. Es besteht die Möglichkeit, dass wenn Benutzer unterschiedlicher Stärkeklassen gegeneinander antreten, der stärkere Spieler ein Handicap erhält oder der schwächere Spieler einen Vorteil erhält.

In einer Ausführungsform kann die gleichzeitige Erfassung von mehreren Benutzern in einem Trainingsmodul und/oder in mehreren Trainingsmodulen erfolgen. Somit können zwei Benutzer in einem Trainingsmodul zusammen agieren oder sie können jeder für sich in voneinander getrennten Trainingsmodulen ihre Übung ausführen. Die unterschiedlichen Trainingsmodule können im gleichen Raum, im gleichen Gebäude oder an ganz unterschiedlichen Orten aufgestellt sein. Die Trainingsmodule können in einem Netzwerk miteinander verbunden sein, beispielsweise können sie über das Internet miteinander verbunden sein. Dementsprechend ist es möglich, dass Benutzer von unterschiedlichen Kontinenten miteinander Übungen ausführen oder gegeneinander antreten.

Die erwähnten Ausführungsformen des Trainingssystems lassen sich in beliebiger Kombination einsetzen, sofern sie sich nicht widersprechen.

Ein erfindungsgemässes Verfahren zum Betreiben eines Trainingsmoduls umfasst die Schritte:
- Bereitstellen eines Trainingsmoduls gemäss einer der vorangehenden Ausführungsformen;
- Auswählen einer Übung durch einen Benutzer;
- Starten der Übung durch die Steuereinheit;
- Erfassen zumindest der Position der Hände des Benutzers mittels der mindestens einen Positionserfassung;
- Vergleichen der erfassten Position mit der in der Steuereinheit hinterlegten Position;
- Anzeigen von Korrekturangaben auf der bildanzeigenden Wand aufgrund der Differenz zwischen der hinterlegten Position und der erfassten Position.

In einer Ausführungsform umfasst das Verfahren zum Betreiben eines Trainingsmoduls mindestens einen der Schritte:
- Anpassen der Übung aufgrund der Auswertung der Differenz zwischen den hinterlegten Positionen und den entsprechenden erfassten Positionen über einen vorgegebenen Zeitraum;
- Anpassen der Übung aufgrund des gemessenen Herzrhythmus über einen vorgegebenen Zeitraum.

### KURZE BESCHREIBUNG DER FIGUREN

Ausführungsbeispiele der vorliegenden Erfindung werden nachstehend anhand von Figuren noch näher erläutert. Diese dienen lediglich zur Erläuterung und sind nicht einschränkend auszulegen. Es zeigen
Fig. 1 eine perspektivische Darstellung eines erfindungsgemässen interaktiven Trainingsmoduls;
Fig. 2 eine perspektivische Darstellung des Gerüstes der Figur 1; und
Fig. 3 eine Teilschnittansicht durch den Aufbau einer Wand der Figur 1.

### DETAILLIERTE BESCHREIBUNG DER ERFINDUNG

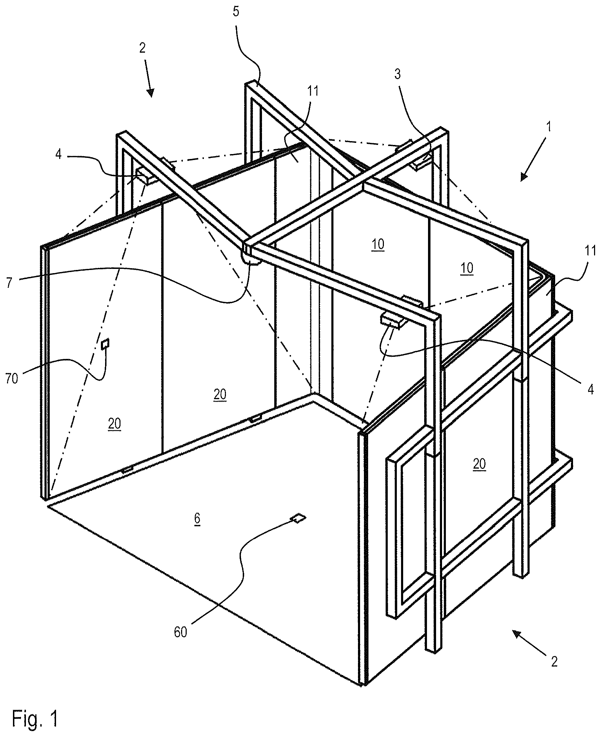

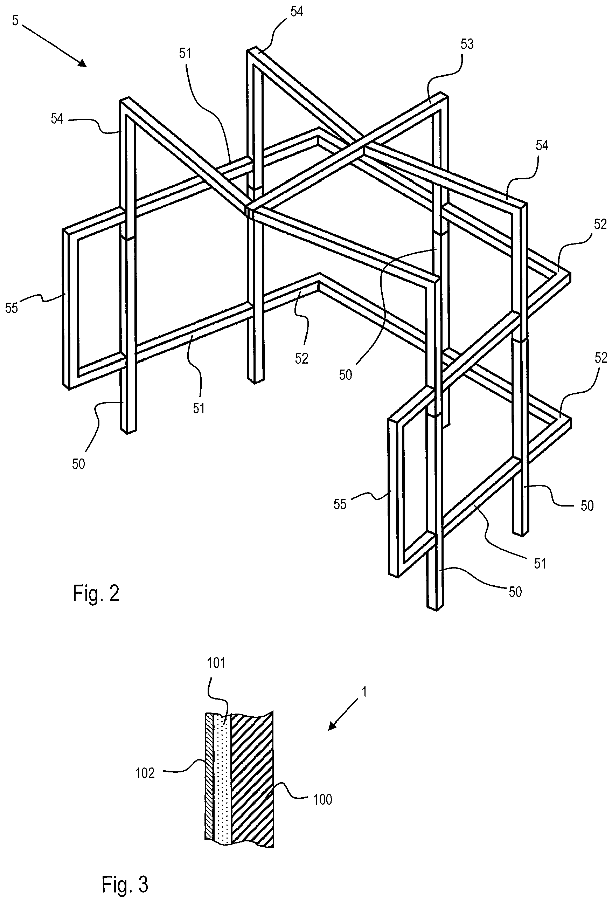

Die Figur 1 zeigt eine perspektivische Darstellung eines erfindungsgemässen interaktiven Trainingsmoduls. Das Trainingsmodul umfasst eine bildanzeigende erste Wand 1 und zwei seitlich anschliessend an diese angeordnete bildanzeigende zweite Wände 2, welche an einem Gerüst 5 angeordnet sind. Die zweiten Wände 2 sind unter einem Winkel von mehr als 90 Grad bezüglich der ersten Wand 1 angeordnet. In der dargestellten Ausführungsform beträgt der zwischen den beiden Wänden 1,2 eingeschlossene Winkel etwa 100 Grad. Die erste Wand 1 umfasst zwei Paneele 10 und jede der zweiten Wände 2 umfasst zwei Paneele 20, welche stumpf aneinander anschliessen. Zwischen der ersten Wand 1 und den zweiten Wänden 2 sind Eckstücke 11 angeordnet, welche auf ihrer einen Seite stumpf an die erste Wand 1 anschliessen und welche an ihrer zweiten Seite stumpf an die zweiten Wände 2 anschliessen. Die Eckstücke sind gerundet ausgebildet, wodurch sich ein im Wesentlichen stufenloser Übergang von der ersten Wand 1 zu den zweiten Wänden 2 ergibt. An den ersten und zweiten Wänden 1,2 sind Kraftsensoren 70 angeordnet. Zur Illustration ist nur ein Kraftsensor 70 angezeigt. Es können jedoch mehrere Kraftsensoren verteilt über die Paneele 10,20 der Wände 1,2 vorgesehen sein. Zwischen den Wänden 1,2 ist ein Boden 6 vorgesehen, welcher ebenfalls mit Kraftsensoren 60 bestückt sein kann. Zur Illustration ist wiederum nur ein Kraftsensor 60 dargestellt. Das Gerüst 5 umfasst mehrere Profile, wie dies in der Figur 2 detailliert dargestellt ist. Die vertikalen Profile 50 ragen über die Wände 1,2 und sind durch Deckenprofile 53,54 miteinander verbunden. An den Deckenprofilen 53,54 sind die Projektoren 3,4 und die Positions-, bzw. die Bewegungserfassung 7 angeordnet. Zur Illustration ist nur ein Bewegungssensor 7 angeordnet.

Die Figur 2 zeigt eine perspektivische Darstellung des Gerüstes 5 der Figur 1. Das Gerüst 5 umfasst fünf Vertikalprofile 50, wobei das mittlere Vertikalprofil 50 der ersten Wand 1 zugeordnet ist und wobei jeweils zwei seitliche Vertikalprofile 50 den zweiten Wänden 2 zugeordnet sind. Am Vertikalprofil 50, welches der ersten Wand 1 zugeordnet ist, ist am oberen Ende ein erstes Deckenprofil 53 angeordnet, welches sich von der ersten Wand 1 weg im Wesentlichen horizontal zwischen die beiden zweiten Wände 2 erstreckt. An den Vertikalprofilen 50, welche den zweiten Wänden 2 zugeordnet sind, ist am jeweiligen oberen Ende ein zweites Deckenprofil 54 angeordnet, welches sich von der jeweiligen zweiten Wand 2 weg im Wesentlichen horizontal bis zum ersten Deckenprofil 53 erstreckt. Die Vertikalprofile 50 der zweiten Wände 2 sind durch erste Horizontalprofile 51 miteinander verbunden und die zweiten Deckenprofile 54 sind ebenfalls mit ersten Horizontalprofilen 51 miteinander verbunden. Am freien Ende der zweiten Wände 2 sind das Vertikalprofil 50 und das zweite Deckenprofil 54 durch ein U-förmiges Verbindungsprofil 55 miteinander verbunden, wobei die Schenkel des Verbindungsprofils 55 fluchtend mit den ersten Horizontalprofilen 51 ausgerichtet sind. Auf der gegen die erste Wand 1 hin gerichteten Seite jeder zweiten Wand 2 sind die Vertikalprofile 50 durch winklige zweite Horizontalprofile 52 miteinander verbunden und das erste Deckenprofil 53 ist beidseitig mit den zweiten Deckenprofilen 54 ebenfalls durch die winkligen zweiten Horizontalprofile 52 verbunden. Jeweils ein Schenkel der zweiten Horizontalprofile 52 ist fluchtend mit jeweils den zweiten Horizontalprofilen 51 ausgerichtet.

Die Figur 3 zeigt eine Teilschnittansicht durch den Aufbau einer Wand 1,2 der Figur 1. Jedes der Paneele 10,20 und jedes Eckprofil 11 weist denselben Schichtaufbau auf. Der Schichtaufbau umfasst eine erste Schicht 100 in der Form einer MDF-Platte, eine an dieser angeordneten zweiten Schicht 101 in der Form einer Schaumstoff-Platte und an dieser angeordneten dritten Schicht 102 in der Form einer Leinwand. Die Schaumstoffplatte 101 ist auf die MDF-Platte aufgeklebt und die Leinwand ist über die Schaumstoffplatte und die MDF Platte gespannt.

**BEZUGSZEICHENLISTE**

| | | | |
|---|---|---|---|
| 1 | erste Wand | 51 | erstes Horizontalprofil |
| 10 | Paneel | | |
| 11 | Eckstück | 52 | zweites Horizontalprofil |
| 100 | erste Schicht | 53 | erstes Deckenprofil |
| 101 | zweite Schicht | 54 | zweites Deckenprofil |
| 102 | dritte Schicht | 55 | Verbindungsprofil |
| 2 | zweite Wand | 6 | Boden |
| 20 | Paneel | 60 | Kraftsensor |
| 3 | erster Projektor | 7 | Bewegungssensor |
| 4 | zweiter Projektor | 70 | Kraftsensor |
| 5 | Gerüst | | |
| 50 | Vertikalprofil | | |

## Patentansprüche

1. Ein interaktives Trainingsmodul umfassend eine bildanzeigende erste Wand (1), zwei bildanzeigende zweite Wände (2), welche auf beiden Seiten der bildanzeigenden ersten Wand (1) seitlich an diese anschliessen, drei Projektoren (3,4), wobei mit jeweils einem der Projektoren (3,4) auf jeweils einer der bildanzeigenden Wände (1,2) einem Benutzer Bewegungsabläufe und/oder Objekte angezeigt werden können, mindestens eine Positionserfassung (7), mit welcher die Positionen zumindest der Hände des Benutzers erfassbar sind und eine Steuereinheit, mit welcher diese Positionen mit denen verglichen werden kann, welche in der Steuereinheit hinterlegt sind, wobei die Steuereinheit aufgrund der Differenz zwischen der hinterlegten Position und der erfassten Position dem Benutzer mittels der mindestens einen bildanzeigenden Wand (1,2) Korrekturangaben anzeigen kann, wobei die bildanzeigende erste Wand (1) und jede der beiden bildanzeigenden zweiten Wände (2) auf der gegen den Benutzer gerichteten Seite jeweils einen Winkel von mehr als 90 Grad einschliessen, wobei die bildanzeigenden Wände (1,2) an einem Gerüst (5) angeordnet sind, welches in der bestimmungsgemässen Gebrauchslage nach oben über die bildanzeigenden Wände (1,2) ragt und wobei die Projektoren (3,4) an dem über die Wände (1,2) ragenden Teil des Gerüsts (5) angeordnet sind.

2. Das interaktive Trainingsmodul gemäss Anspruch 1, wobei die Positionserfassung (7) mindestens eine Kamera umfasst und/oder wobei die Positionserfassung (7) mindestens einen induktiven, kapazitiven oder Infrarotlicht erfassenden Sensor umfasst, mit welchem der Bereich vor der Wand (1,2) erfassbar ist.

3. Das interaktive Trainingsmodul gemäss Anspruch 1 oder 2, umfassend mindestens einen Lautsprecher, mit welchem Instruktionen gegeben werden können und/oder mit welchem akustische Korrekturangaben gemacht werden können und/oder mit welchem motivierende oder animierende Kommentare oder Musik abgegeben werden können.

4. Das interaktive Trainingsmodul gemäss einem der vorangehenden Ansprüche, umfassend mindestens einen Herzrhythmus-Sensor, mit welchem der Herzrhythmus des Benutzers kontinuierlich und/oder in Intervallen erfassbar ist, wobei die Steuereinheit den erfassten Herzrhythmus verwenden kann, um den Schwierigkeitsgrad und/oder die Intensität des Trainingsprogramms anzupassen.

5. Das interaktive Trainingsmodul gemäss einem der vorangehenden Ansprüche, umfassend einen Kraftsensor (70), welcher in der mindestens einen bildanzeigenden Wand (1,2) integriert ist.

6. Das interaktive Trainingsmodul gemäss einem der vorangehenden Ansprüche, wobei die mindestens eine bildanzeigende Wand (1,2) eine tragende erste Schicht (100) und eine haptische zweite Schicht (101) umfasst, welche auf der gegen den Benutzer gerichteten Seite der ersten Schicht (100) angeordnet ist, und eine optische dritte Schicht (102) umfasst, welche auf der gegen den Benutzer gerichteten Seite der zweiten Schicht (101) angeordnet ist.

7. Das interaktive Trainingsmodul gemäss Anspruch 6, wobei die erste Schicht (100) eine MDF-Platte umfasst und wobei die zweite Schicht (101) eine Schaumstoff-Platte, eine Silikon-Platte oder eine Silikon-Schaumstoff-Platte umfasst, welche mit der MDF-Platte (100) verleimt ist.

8. Das interaktive Trainingsmodul gemäss Anspruch 6, wobei die dritte Schicht (102) eine Leinwand umfasst, welche zumindest über die Schaumstoff-Platte, die Silikon-Platte oder die Silikon-Schaumstoff-Platte (101) gespannt ist.

9. Das interaktive Trainingsmodul gemäss einem der vorangehenden Ansprüche, wobei die Positionserfassung (7) an dem über die Wände (1,2) ragenden Teil des Gerüsts (5) angeordnet ist.

10. Das interaktive Trainingsmodul gemäss einem der vorangehenden Ansprüche, umfassend einen Boden (6), welcher bildanzeigend ausgestaltet sein kann und/oder welcher mit Kraftsensoren (60) bestückt ist und umfassend mindestens einen Projektor, mit welchem ein Bild auf dem Boden (6) anzeigbar ist.

11. Ein interaktives Trainingssystem umfassend ein Trainingsmodul gemäss einem der vorangehenden Ansprüche und mindestens einen Tracker, welcher von einem Benutzer tragbar ist und mit welchem zumindest die Position einer Hand des Benutzers erfassbar ist.

12. Das interaktive Trainingssystem gemäss Anspruch 11, wobei das Trainingssystem derart ausgebildet ist, dass die Bewegungen von zwei oder mehr Benutzern erfassbar sind.

13. Das interaktive Trainingssystem gemäss Anspruch 12, wobei die Erfassung gleichzeitig erfolgen kann.

14. Das interaktive Trainingssystem gemäss Anspruch 12 oder 13, wobei die gleichzeitige Erfassung von mehreren Benutzern in einem Trainingsmodul und/oder in mehreren Trainingsmodulen erfolgen kann.

15. Ein Verfahren zum Betreiben eines Trainingsmoduls umfassend die Schritte:
- Bereitstellen eines Trainingsmoduls gemäss einem der Ansprüche 1 bis 10;
- Auswählen einer Übung durch einen Benutzer;
- Starten der Übung durch die Steuereinheit;
- Erfassen zumindest der Position der Hände des Benutzers mittels der mindestens einen Positionserfassung (7) ;
- Vergleichen der erfassten Position mit der in der Steuereinheit hinterlegten Position;
- Anzeigen von Korrekturangaben auf der bildanzeigenden Wand (1,2) aufgrund der Differenz zwischen der hinterlegten Position und der erfassten Position.

## Claims

1. An interactive training module comprising at least one image-displaying first wall (1), two image-displaying second walls (2) that are laterally connected to both sides of the image-displaying first wall (1), three projectors (3,4), wherein motion sequences and/or objects can be displayed to a user with one of the projectors (3,4) each on one of the image-displaying walls (1,2), at least one position sensing device (7) with which the positions of at least the user's hands can be detected and a control unit with which these positions can be compared with those which are stored in the control unit, wherein the control unit can display correction information to the user by means of the at least one image-displaying wall (1,2) based on the difference between the stored position and the detected position, wherein the image-displaying first wall (1) and each of the two image-displaying second walls (2) facing the user include an angle of at least 90 degree, wherein the image-displaying walls (1,2) are arranged on a framework (5) that projects upwardly above the image-displaying walls (1,2) in the intended use position and wherein the projectors (3,4) are arranged on the part of the framework (5) projecting above the walls (1,2).

2. The interactive training module according to claim 1, wherein the position sensing device (7) comprises at least one camera and/or wherein the position sensing device (7) comprises at least one inductive, capacitive or infrared light detecting sensor with which the area in front of the wall (1,2) is detectable.

3. The interactive training module according to claim 1 or 2, comprising at least one loudspeaker with which instructions can be given and/or with which acoustic correction indications can be made and/or with which motivating or animating comments or music can be provided.

4. The interactive module according to one of the preceding claims, comprising at least one heart rhythm sensor with which the heart rhythm of the user can be detected continuously and/or at intervals, wherein the control unit can use the sensed heart rhythm to adjust the difficulty and/or the intensity of the training program.

5. The interactive training module according to one of the preceding claims, comprising a force sensor (70) integrated in the at least one image-displaying wall (1,2).

6. The interactive training module according to one of the preceding claims, wherein the at least one image-displaying wall (1,2) comprises a supporting first layer (100) and a haptic second layer (101) arranged on the side of the first layer (100) facing the user, and an optical third layer (102) arranged on the side of the second layer (101) facing the user.

7. The interactive training module according to claim 6, wherein the first layer (100) comprises an MDF board and wherein the second layer (101) comprises a foam board, a silicone board or a silicon foam board glued to the MDF board (100) .

8. The interactive training module according to claim 6, wherein the third layer (102) comprises a canvas stretched over at least the foam board, the silicone board or the silicone foam board (101).

9. The interactive training module according to one of the preceding claims, wherein the position sensing device (7) is arranged on the part of the framework (5) projecting above the walls (1,2).

10. The interactive training module according to one of the preceding claims, comprising a floor (6) which may be image-displaying and/or which is equipped with force sensors (60) and comprising at least one projector with which an image on the floor is displayable.

11. An interactive training system, comprising a training module according to one of the preceding claims and at least one tracker which is wearable by a user and with which the position of a hand of the user is detectable.

12. The interactive training system according to claim 11, wherein the training system is configured such that the movements of two or more users are detectable.

13. The interactive training system according to claim 12, wherein the detection may be simultaneous.

14. The interactive training system according to claim 12 or 13, wherein simultaneous detection of multiple users can be performed in one training module and/or multiple training modules.

15. A method for operating a training module comprising the steps of:
- providing a training module according to any one of claims 1 to 10;
- selecting an exercise by a user;
- starting the exercise by the control unit;
- detecting at least the position of the user's hands by means of the at least one position sensing device (7);
- comparing the detected position with the position stored in the control unit;
- displaying correction information on the image-displaying wall (1,2) based on the difference between the deposited position and the detected position.

## Revendications

1. Module d'entraînement interactif comprenant au moins une première paroi affichant des images (1), deux deuxièmes parois (2) affichant des images et reliées latéralement aux deux côtés de la première paroi (1) affichant des images, trois projecteurs (3,4), dans lesquels des séquences de mouvement et/ou des objets peuvent être affichés à un utilisateur avec l'un des projecteurs (3,4) sur l'une des parois (1,2) affichants des images, au moins un dispositif de détection de position (7), avec laquelle les positions d'au moins les mains de l'utilisateur peuvent être détectées et une unité de commande permettant de comparer ces positions avec celles qui sont enregistrées dans l'unité de commande, l'unité de commande pouvant, sur la base de la différence entre la position enregistrée et la position détectée, indiquer à l'utilisateur des indications de correction au moyen de la au moins une paroi (1,2) affichant des images, dans lequel la première paroi affichant des images (1) et chacune des deux deuxièmes parois affichant des images (2) forment un angle de plus de 90 degrés sur le côté faisant face à l'utilisateur, respectivement, **caractérisé en ce que** les parois affichant des images (1,2) sont disposées sur une charpente (5) qui, dans la position d'utilisation prévue, dépasse vers le haut au-dessus des parois affichant des images (1,2) et **en ce que** les projecteurs (3,4) sont disposés sur la partie de la charpente (5) dépassant des parois (1,2).

2. Le module d'entraînement interactif selon la revendication 1, dans lequel le dispositif de détection de position (7) comprend au moins une caméra et/ou dans lequel le dispositif de détection de position (7) comprend au moins un capteur inductif, capacitif ou de détection de lumière infrarouge, avec lequel la zone devant la paroi (1,2) peut être détectée.

3. Le module d'entraînement interactif selon la revendication 1 ou 2, comprenant au moins un haut-parleur avec lequel des instructions peuvent être données et/ou avec lequel des indications de correction acoustiques peuvent être données et/ou avec lequel des commentaires ou de la musique motivants ou animants peuvent être émis.

4. Le module d'entraînement interactif selon l'une des revendications précédentes, comprenant au moins un capteur de rythme cardiaque permettant de détecter en continu et/ou par intervalles le rythme cardiaque de l'utilisateur, l'unité de commande pouvant utiliser le rythme cardiaque détecté pour adapter le niveau de difficulté et/ou l'intensité du programme d'entraînement.

5. Le module d'entraînement interactif selon l'une des revendications précédentes, comprenant un capteur de force (70) intégré dans l'au moins une paroi affichant des images (1,2) .

6. Le module d'entraînement interactif selon l'une des revendications précédentes, dans lequel l'au moins une paroi affichant des images (1,2) comprend une première couche portante (100) et une deuxième couche haptique (101), qui est disposée sur le côté de la première couche (100) orienté vers l'utilisateur, et une troisième couche optique (102), qui est disposée sur le côté de la deuxième couche (101) orienté vers l'utilisateur.

7. Le module d'entraînement interactif selon la revendication 6, dans lequel la première couche (100) comprend un panneau MDF et dans lequel la deuxième couche (101) comprend un panneau de mousse, un panneau de silicone ou un panneau de mousse de silicone qui est collé au panneau MDF (100).

8. Le module d'entraînement interactif selon la revendication 6, dans lequel la troisième couche (102) comprend une toile tendue au moins sur la plaque de mousse, la plaque de silicone ou la plaque de mousse de silicone (101).

9. Le module d'entraînement interactif selon l'une des revendications précédentes, dans lequel le dispositif de détection de position (7) est disposée sur la partie de la charpente (5) dépassant des parois (1,2).

10. Le module d'entraînement interactif selon l'une des revendications précédentes, comprenant un sol (6), qui peut être conçu pour afficher une image et/ou qui est équipé de capteurs de force (60) et comprenant au moins un projecteur, avec lequel une image peut être affichée sur le sol (6).

11. Système d'entraînement interactif comprenant un module d'entraînement selon l'une des revendications précédentes et au moins un tracker, qui peut être porté par un utilisateur et avec lequel au moins la position d'une main de l'utilisateur peut être détectée.

12. Le système d'entraînement interactif selon la revendication 11, dans lequel le système d'entraînement est conçu de telle sorte que les mouvements de deux utilisateurs ou plus peuvent être détectés.

13. Le système d'entraînement interactif selon la revendication 12, dans lequel la détection peut être simultanée.

14. Le système d'entraînement interactif selon la revendication 12 ou 13, dans lequel la détection simultanée de plusieurs utilisateurs peut être effectuée dans un module d'entraînement et/ou dans plusieurs modules d'entraînement.

15. Procédé d'exploitation d'un module d'entraînement comprenant les étapes suivantes :
- Fournir un module d'entraînement selon l'une des revendications 1 à 10 ;
- Sélection d'un exercice par un utilisateur ;
- Démarrage de l'exercice par l'unité de commande ;
- Détection d'au moins la position des mains de l'utilisateur au moyen de l'au moins un disposirif de détection de position (7) ;
- Comparaison de la position détectée avec la position enregistrée dans l'unité de commande ;
- Affichage d'indications de correction sur la paroi affichant des images (1, 2) sur la base de la différence entre la position enregistrée et la position détectée.
